(19) Europäisches Patentamt
European Patent Office
Office européen des brevets

(11) EP 1 307 427 B3

(12) **NEW EUROPEAN PATENT SPECIFICATION**
After limitation procedure (B3-1)

(45) Date of publication and mention of the limitation decision:
**B3-1 29.04.2009 Bulletin 2009/18**

(45) Mention of the grant of the patent:
**12.04.2006 Bulletin 2006/15**

(21) Application number: **01953252.2**

(22) Date of filing: **01.08.2001**

(51) Int Cl.:
***C07C 253/00*** *(2006.01)*

(86) International application number:
**PCT/GB2001/003456**

(87) International publication number:
**WO 2002/010095 (07.02.2002 Gazette 2002/06)**

(54) **PROCESS FOR THE CYANATION OF ALDEHYDES**

VERFAHREN ZUR CYANIERUNG VON ALDEHYDEN

PROCEDE DE PRODUCTION DE CYANHYDRINES A PARTIR D'ALDEHYDES

(84) Designated Contracting States:
**AT BE CH CY DE DK ES FI FR GB GR IE IT LI LU MC NL PT SE TR**

(30) Priority: **02.08.2000 GB 0018973**

(43) Date of publication of application:
**07.05.2003 Bulletin 2003/19**

(73) Proprietors:
- **KING'S COLLEGE LONDON**
  **London WC2R 2LS (GB)**
- **Nesmeyanov Institute of Organoelement Compounds**
  **Moscow (RU)**

(72) Inventors:
- **NORTH, Michael**
  **London WC2R 2LS (GB)**
- **BELOKON, Yuri**
  **Moscow (RU)**

(74) Representative: **Atkinson, Jonathan David Mark**
**Harrison Goddard Foote**
**Belgrave Hall**
**Belgrave Street**
**Leeds**
**LS2 8DD (GB)**

(56) References cited:
**EP-A- 0 561 637** **US-A- 4 336 206**

- **PATENT ABSTRACTS OF JAPAN vol. 1995, no. 06, 31 July 1995 (1995-07-31) -& JP 07 070040 A (SUMITOMO CHEM CO LTD), 14 March 1995 (1995-03-14)**
- **PATENT ABSTRACTS OF JAPAN vol. 018, no. 680 (C-1291), 21 December 1994 (1994-12-21) -& JP 06 271522 A (SUMITOMO CHEM CO LTD), 27 September 1994 (1994-09-27)**
- **Y.N. BELOKON E.A.: "The asymmetric Addition of trimethylsilyl cyanide to aldehydes catalyzed by chiral (salen)titanium complexes" JOURNAL OF THE AMERICAN CHEMICAL SOCIETY., vol. 121, 1999, pages 3968-3973, XP002187021 DC US cited in the application**



Printed by Jouve, 75001 PARIS (FR)

## Description

This invention relates to a process for the cyanation of aldehydes, particularly to the asymmetric cyanation of aldehydes, including the synthesis of chiral cyanohydrins and derivatives thereof, such as chiral O-acyl cyanohydrins.

The synthesis of chiral intermediates such as chiral cyanohydrins and derivatives is an important process for use in the manufacture of fine chemicals, agrochemicals and pharmaceuticals. Enantiomerically pure cyanohydrins and derivatives are known to be versatile intermediates for the synthesis of a wide range of commercially important compounds. For example chiral cyanohydrins and derivatives are intermediates for the synthesis of: α-hydroxy-acids, a-amino alcohols, and 1,2-diols. In addition, chiral cyanohydrins are themselves components of highly successful pyrethroid insecticides.

There are a number of synthetic routes available for the asymmetric synthesis of cyanohydrins and derivatives, virtually all of which involve the use of a chiral catalyst to induce the asymmetric addition of a cyanide source to a prochiral aldehyde or ketone. The available catalysts include enzymes, cyclic peptides and transition metal complexes. However, all of these methods suffer from one or more significant disadvantages which have negated their commercial exploitation. Many of the methods employ highly toxic and hazardous HCN, require very low (ca. -80°C) reaction temperatures, and/or give products with low to moderate enantiomeric excesses.

Processes for the asymmetric synthesis cyanohydrins and derivatives are disclosed by M.North, Synlett, 1993, 807-20; F.Effenberger, Angew. Chem. Int. Ed, Engl.1994, 33, 1555; M.North, Comprehensive Organic Functional Group Transformations ed. Katritzky, A.R.; Meth-Cohn, O.; Rees, C.W.; Pattenden. G.; Pergamon Press, Oxford, 1995, vol. 3, chapter 18: Y Belokon' et al, Tetrahedron Asymmetry, 1996, 7, 851-5; Y.Belokon' et al,J.Chem. Soc., Perkin Trans. 1, 1997, 1293-5; Y.N.Belokon' et al, Izvestiya Akademii Nauk. Seriya Khimicheskaya, 1997, 2040: translated as Russian Chem. Bull., 1997, 46, 1936-8; V.I.Tararov et al, Chem. Commun., 1998, 387-8; Y.N.Belokon' et al. J. Am. Chem. Soc., 1999, 121, 3958-73; V.I.Tararov et al, Russ. Chem. Bull., 1999, 48, 1128-30; Y. N.Belokon' et al, Tetrahedron Lett., 1999, 40, 8147-50; Y.N.Belokon' et al, Eur. J. Org. Chem., 2000, 2655-61; Y.N.Betokon', M.North, and T.Parsons; Org. Lett., 2000, 2, 1617-9; and in JP07 070040 (Sumitomo Chem Co Ltd) 14 March 1995; and JP06 271522 (Sumitomo Chem Co Ltd) 27 September 1994.

In particular J. Am. Chem. Soc., 1999, 121, 3968-73 discloses the use of catalysts 1 and 2 haying the formulae given below (with $R^1$ and $R^2$ = tert-butyl) which are the most active catalysts known for this reaction (Scheme 1).

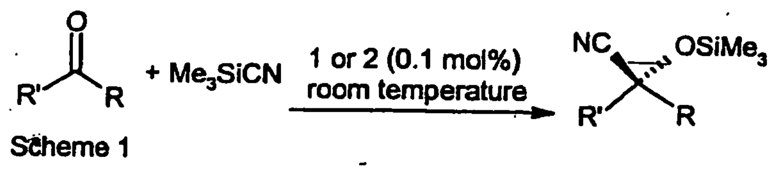


Scheme 1

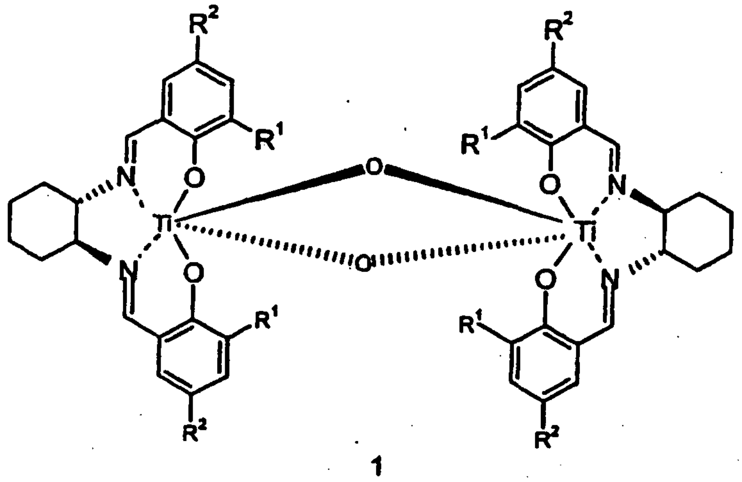

1

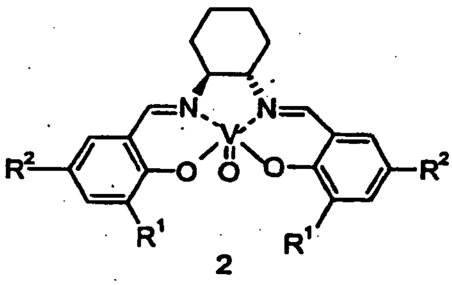

2

wherein each $R^1$ and $R^2$ independently is H, alkyl, aryl, aralkyl, alkoxy, aryloxy, halogen, nitro, halo-alkyl, amino (including with alkyl or aryl substituents on the nitrogen atom), or amido. Preferably, $R^1$ and $R^2$ = $CMe_3$. However, whilst the chemistry shown in Scheme 1 is academically interesting, it is of little commercial relevance due to the prohibitive cost of trimethylsilyl cyanide. Additionally, trimethylsilyl cyanide is highly volatile and hazardous to handle.

According to a first aspect of the present invention, there is provided a process for cyanating an aldehyde which comprises reacting the aldehyde with: i) a cyanide source which does not comprise a Si-CN or a C-(C=O)-CN moiety; and ii) a substrate having the general formula Q-Y where Q represents an organic acid radical selected from the group comprising: R-(C=O)-, R-(C=S)-, RO-(C=O)-, RN-(C=O)-,RO-(C=S)-, RN-(C=S)-, RS-(C=O)-, RS-(C=S)-, R-(P=O)(OR)-, $R-SO_2-$ and R-SO-, wherein R represents a substituted or unsubstituted hydrocarbyl group; and Y represents a non-halogen leaving group; in the presence of a catalyst comprising a chiral complex of titanium or vanadium.

Aldehydes which can be employed in the process of the present invention have the chemical formula R-CHO, wherein R is a substituted or unsubstituted hydrocarbyl group, including perhalogenated hydrocarbyl groups. Hydrocarbyl groups which may be represented by R include alkyl, alkenyl, aryl and heterocyclic groups, and any combination thereof, such as aralkyll and alkaryl, for example benzyl groups.

Alkyl groups which may be represented by R include linear and branched alkyl groups comprising up to 20 carbon atoms, particularly from 1 to 7 carbon atoms and preferably from 1 to 5 carbon atoms. When the alkyl groups are branched, the groups often comprise up to 10 branched chain carbon atoms, preferably up to 4 branched chain atoms. In certain embodiments, the alkyl group may be cyclic, commonly comprising from 3 to 10 carbon atoms in the largest ring and optionally featuring one or more bridging rings. Examples of alkyl groups which may be represented by R include methyl, ethyl, propyl, 2-propyl, butyl, 2-butyl, t-butyl and cyclohexyl groups.

Alkenyl groups which may be represented by R include $C_{2-20}$, and preferably $C_{2-6}$ alkenyl groups. One or more carbon - carbon double bonds may be present. The alkenyl group may carry one or more substituents, particularly phenyl substituents. Examples of alkenyl groups include vinyl, styryl and indenyl groups.

Aryl groups which may be represented by R may contain 1 ring or 2 or more fused rings which may include cycloalkyl, aryl or heterocyclic rings. Examples of aryl groups which may be represented by R include phenyl, tolyl, fluorophenyl, chlorophenyl, bromophenyl, trifluoromethylphenyl, anisyl, naphthyl and ferrocenyl groups.

Perhalogenated hydrocarbyl groups which may be represented by R include perhalogenated alkyl and aryl groups, and any combination thereof, such as aralkyl and alkaryl groups. Examples of perhalogenated alkyl groups which may be represented by R include $-CF_3$ and $-C_2F_5$.

Heterocyclic groups which may be represented by R include aromatic, saturated and partially unsaturated ring systems and may constitute 1 ring or 2 or more fused rings which may include cycloalkyl, aryl or heterocyclic rings. The heterocyclic group will contain at least one heterocyclic ring, the largest of which will commonly comprise from 3 to 7 ring atoms in which at least one atom is carbon and at least one atom is any of N, O, S or P. Examples of heterocyclic groups which may be represented by R include pyridyl, pyrimidyl, pyrrolyl, thiophenyl, furanyl, indolyl, quinolyl, isoquinolyl, imidazoyl and triazoyl groups.

When R is a substituted hydrocarbyl or heterocyclic group, the substituent(s) should be such so as not to adversely affect the reaction. Optional substituents include halogen, cyano, nitro, hydroxy, amino, thiol, acyl, hydrocarbyl, perhalogenated hydrocarbyl, heterocyclyl, hydrocarbyloxy, mono or di-hydrocarbylamino, hydrocarbylthio, esters, carbonates, amides, sulphonyl and sulphonamido groups wherein the hydrocarbyl groups are as defined for R above. One or more substituents may be present.

Cyanide sources not comprising a Si-CN bond or a C-(C=O)-CN moiety which can be employed in the process of the present invention include dicyanogen; ammonium cyanide salts, particularly quaternary ammonium salts such as tetraalkyl, preferably tetra $C_{1-6}$alkyl-, ammonium salts; sulfonyl cyanides, for example tosyl cyanide and mesyl cyanide; and organic cyanides having the formula $R^3$-O-CO-CN, where $R^3$ is H or a substituted or unsubstituted hydrocarbyl group as described above, commonly a $C_{1-6}$ alkyl group. In many embodiments, the cyanide source is an inorganic cyanide, preferably a metal cyanide or an in situ source of inorganic cyanide such as acetone cyanohydrin. Particularly preferred cyanide sources comprise alkali metal and alkaline earth metal cyanides, for example, lithium, sodium, potassium, rubidium, caesium, magnesium and calcium cyanides. The most preferred cyanide source is potassium cyanide.

The reaction between the aldehyde and the cyanide source occurs in the presence of a substrate susceptible to nucleophilic attack which does not comprise a halogen leaving group. Examples of such substrates are compounds having the general formula Q-Y, wherein Q represents an organic acid radical, and Y represents a non-halogen leaving group. In many embodiments, the leaving group, Y, is a leaving group the conjugate acid of which has a pKa of greater than about -2, such as greater than 3, and often less than 12. Examples of leaving groups include alkyl and aryl sulphonates, such as mesylate and tosylate; carbonates; especially alkyl carbonates; carboxylates, especially alkyl carboxylates; and groups of formula $-NR^xR^y$, wherein $R^x$ and $R^y$ together with the nitrogen atom form an unsaturated heterocyclic ring which may comprise one ormore additional heteroatoms, especially nitrogen, particularly imidazole or benzimidazole rings. Organic acid radicals which may be represented by Q include groups of formulae R-(C=O)-, R-(C=S)-, RO-(C=O)-, RN-(C=O)-, RO-(C=S)-, RN-(C=S)-, RS-(C=O)-, RS-(C=S)-, R-(P=O)(OR)-, $R-SO_2$- and R-SO-, wherein R represents a substituted or unsubstituted hydrocarbyl group as described above.

In many embodiments, the substrate susceptible to nucleophilic attack which does not comprise a halogen leaving group has the general formula $R^4$-(C=X)-A-Z, wherein $R^4$ represents an organic radical, such as a substituted or unsubstituted hydrocarbyl group as described above or a hydrocarbyloxy group wherein the hydrocarbyl moiety is as described above; X represents O, S, N-R or NOR wherein R represents H or a substituted or unsubstituted hydrocarbyl group as described above; A represents a chalcogen, preferably O or S and Z represents a group of formula (C=O)-$R^4$ or (C=S)-$R^4$ wherein $R^4$ is as described above; or -A-Z represents a group of formula $-NR^xR^y$ as described above. Preferably, X and A each represent O, and Z is a group of formula (C=O)-$R^4$.

Commonly, the substrate susceptible to nucleophilic attack which does not comprise a halogen leaving group is a carboxylic acid anhydride or an anhydride of a carbonic acid. Carboxylic anhydrides include mixed anhydrides and are often the anhydrides of $C_{1-8}$ alkyl or aryl carboxylic acids, such as acetic anhydride and trifluoroacetic anhydride. Carbonic acid anhydrides include di-tert-butyldicarbonate, (tBuOCOOCOOtBu), N,N'-disuccinyldicarbonate, N,N'-dimaleimyldicarbonate, N-(tert-butyl-oxycarbonyloxy) maleimide orsuccinimide, and N-(benzyloxycarbonyloxy) maleimide or succinimide.

Chiral catalysts that can be employed in the process of the present invention are those known in the art for catalysing the addition of a cyanide group to a carbonyl group, and include enzymes and cyclic peptides. Preferably, the chiral catalysts are metal complexes of metals, for example B, Mg, Al, Sn, Bi, particularly transition-metal complexes comprising a chiral ligand, for example Re and lanthanides. In many embodiments, the transition metal is a Lewis acid capable of forming tetra coordinate complexes with chiral ligands. Preferred transition metal complexes are complexes of titanium and vanadium, especially titanium (IV) and vanadium (V). The chiral ligands are preferably tetradentate and commonly coordinate via oxygen and/or nitrogen atoms. Examples include binol, taddol, sulfoximines, salicylimines and tartrates, especially tartrate esters. However, the most preferred class of ligands are chiral Salen ligands and derivatives thereof. Particularly preferably, the chiral catalyst employed in the process according to the present invention is a catalyst of formula 1 or 2 described above. When a catalyst of formula 1 is employed, it is also possible to use a mixed catalyst containing one vanadium and one titanium ion in each bimetallic catalyst unit.

The process according to the present invention is commonly carried out in the presence of a solvent. Preferred

solvents are polar, aprotic solvents, including halocarbons, for example dichloromethane, chloroform and 1,2-dichloroethane; nitriles, for example acetonitrile; ketones, for example acetone and methylethylketone; ethers, for example diethylether and tetrahydrofuran; and amides, for example dimethylformamide, dimethylacetamide and N-methylpyrolidinone.

Advantageously, the process of the present invention is carried out in the presence of an additive which accelerates the rate of reaction. Commonly these additives are inorganic bases such as $Na_2CO_3$, $K_2CO_3$ or $CaCO_3$ or comprise a nucleophilic heteroatom, and often have pKa of greater than 10, for example in the range from 15-35, such as from 15-25. Examples of preferred additives include organic bases, such as pyridine, 2,6-lutidine and imidazole; alcohols, such as $C_{1-6}$ alcohols, especially tertiary alcohols such as t-butanol; and water.

It will be recognised thatwhen the cyanide source is a metal cyanide, the reaction mixture will be heterogeneous. In such circumstances, it is therefore desirable to employ efficient agitation of the reaction mixture. Agitation means known in the art, for example mechanical stirrers and ultrasonic agitators, selected appropriately according to the scale of reaction can be employed as desired.

The process of the present invention is often carried out a temperature of from about -40°C to about 40°C. Lower temperatures may be employed if desired, although they are not believed to be advantageous. Commonly, the reaction is carried out a temperature of from -25°C to ambient temperature, such as 15-25°C.

The product of the cyanation reaction in the presence of the substrate susceptible to nucleophilic attack which does not comprise a halogen leaving group can then be reacted, for example by hydrolysis, to form a cyanohydrin. When the substrate susceptible to nucleophilic attackwhich does not comprise a halogen leaving group has the general formula Q-Y, the process can be represented by the sequence:

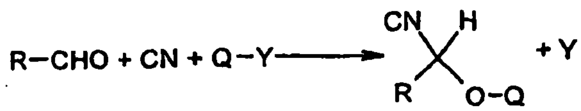

The process according to the present invention is particularly suited to the enantioslective cyanation of aldehydes. It has been found that particularly effective enantioselective cyanation of aldehydes can be achieved by employing an order of addition in which a mixture of chiral catalyst, cyanide source, solvent and aldehyde are prepared, preferably an additive as described above is added to this mixture. The temperature of this mixture is then adjusted to the desired reaction temperature if necessary, and the substrate susceptible to nucleophilic attack not comprising a halogen leaving group is added. This approach has been found to be especially suited when the additive comprises lutidine and the substrate susceptible to nucleophilic attack not comprising a halogen leaving group is a carboxylic anhydride.

Certain embodiments of the present invention comprise the use of a heterogeneous mixture of an alkali metal cyanide, or alkaline earth metal cyanide (or other inexpensive cyanide sources such as acetone cyanohydrin), an additive (which may be a base e.g. pyridine; or water) and acetic anhydride (or other carboxylic acid anhydrides) to generate a cyanating agent for aldehydes. This can be carried out in situ with catalyst 1 (and related catalysts) and an aldehyde to generate chiral O-acyl cyanohydrins (conditions as illustrated in Scheme 2). This methodology uses only inexpensive reagents, and produces cyanohydrin derivatives which are not sensitive to moisture and do not spontaneously racemize.

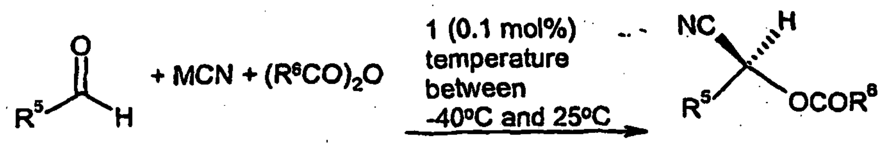


Scheme 2

$R^5$ = alkyl, aryl, aralkyl, and may contain halogen, oxygen, nitrogen, or sulfur atoms within the group. $R^6$ = alkyl, aryl, aralkyl, and may contain halogen, oxygen, nitrogen, or sulfur atoms within the group. M = alkali metal or alkaline earth metal. Preferably, potassium cyanide is used as the cyanide source, acetic anhydride as the anhydride, 2,6-lutidine as the additive and catalyst (or the corresponding enantiomer derived from (R,R-cyclohexane-1,2-diamine) with $R^1$ and $R^2$ = $^tBu$ is used as the catalyst.

This invention allows the synthesis of chiral cyanohydrin derivatives derived from a wide variety of aldehydes. The products can be transformed into other chiral compounds by standard chemistry using either of the acyl or nitrile

functional groups.

According to one preferred aspect of the present invention there is provided a process for the cyanation of an aldehyde group which comprises reacting the aldehyde with:

i) an alkali metal cyanide; and
ii) a carboxylic anhydride;

in the presence of a catalyst comprising a chiral complex of titanium or vanadium.

According to another preferred aspect of the present invention there is provided a process for the preparation of an O-acyl cyanohydrin which comprises reacting an aldehyde with potassium cyanide and a carboxylic anhydride in the presence of a catalyst comprising a chiral complex of titanium or vanadium.

In the preferred aspects, further preferences are as described above with respect to the first aspect of the present invention.

In certain embodiments, the chiral transition metal catalyst and a metal cyanide can be added as mixture. Such a mixture is believed to be a novel composition of matter, and accordingly forms another aspect of the present invention. Preferred transition metal catalysts and metal cyanides are as described above with respect to the first aspect of the present invention.

The invention is illustrated, without limitation, by the following examples. In the examples, catalyst 1 a has the formula:

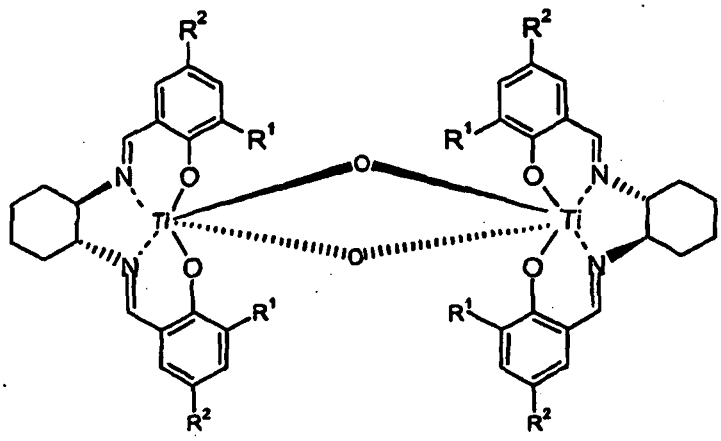

and catalyst 1 b has the formula:

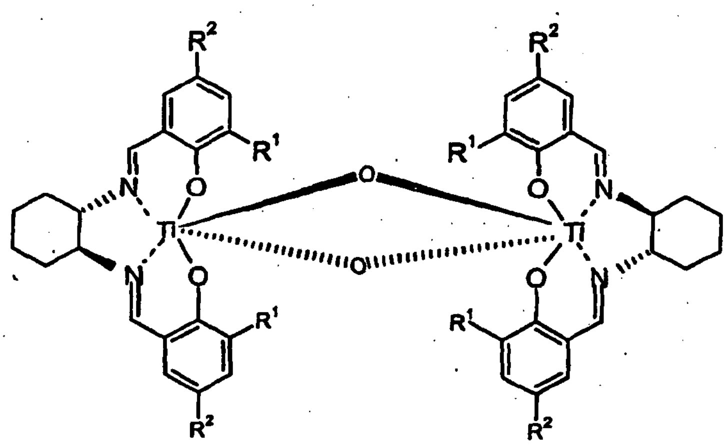

## EXAMPLE 1

To a mixture of KCN (5.0 g, 77 mmol) and catalyst 1a ($R^1 = R^2 = {}^tBu$)(0.3 g, 0.25 mmol) in $CH_2Cl_2$ (60 ml) were added with stirring benzaldehyde (2.5 ml, 25 mmol), 2,6-lutidlne (0.28 ml, 2.4 mmol) and water (0.4 ml, 24 mmol). The reaction mixture was cooled to -30°C and $Ac_2O$ (5 ml, 53 mmol) was added. The reaction mixture was stirred for 10 hours at -30°C and then filtered, passed through a $SiO_2$ column (1 cm x 10 cm) in a mixture of hexane/AcOEt 10:1 to remove the catalyst. The filtrate was evaporated and distilled in vacuo to give 2.7 g (63%) of O-Acetyl (S)-mandelonitrile with 87% enantiomeric excess as determined by chiral gas chromatography.

The experimental procedure was employed with a range of aldehydes under the same conditions, except the reaction temperature. The temperature employed and the results achieved are given in Table 1 below.

**TABLE 1**

| $R^2$ ($R^1$ = H) | Temperature = 24 °C | Temperature = - 40 °C | Temperature = -78 °C |
|---|---|---|---|
| | ee (%) | Ee (%) | ee (%) |
| $C_6H_5$ | 80 | 90 | 86 |
| $4\text{-}CF_3C_6H_4$ | 60 | 76 | 54 |
| $4\text{-}FC_6H_4$ | 65 | 90 | 84 |
| $4\text{-}ClC_6H_4$ | - | 90 | - |
| $2\text{-}FC_6H_4$ | 45 | 86 | 88 |
| $3\text{-}PhOC_6H_4$ | - | 90 | - |
| $C_6H_5CH_2CH_2$ | 40 | 82 | - |
| $(CH_3)_2CH$ | - | 64 | - |
| $(CH_3)_3C$ | 40 | 62 | |

The reactions can be run at room temperature, giving quantitative chemical yields in 2-3 hours or at lower temperatures, the latter giving better enantiomeric excesses, though at the expense of lower chemical yields (40-70%) and longer reaction times (10 hours).

## EXAMPLE 2

The method of Example 1 was repeated for the cyanation of 3-phenyl propanal at -35°C, except that imidazole was employed in place of 2,6-lutidine. The O-acetyl cyanohydrin was obtained in 90% yield and 85% ee.

## EXAMPLE 3

The method of Example 1 was repeated, except that catalyst 1a wherein $R^1$ = Ph, and $R^2$ = H gave a 90% chemical yield and 82% ee when used at -35°C with benzaldehyde as substrate.

## EXAMPLE 4

A stirred mixture of KCN (12.37 g, 0.19 mol), catalyst 1b (0.487 g, $4\times10^{-4}$ mol), *t*-BuOH (3.7 g, 4.8 mL, $5.0\times10^{-2}$ mol) and 2-chlorobenzaldehyde (6.68 g, 5.35 mL, $4.75\times10^{-2}$ mol) in dry dichloromethane (120 mL) was cooled to -42°C, and acetic anhydride (19.4 g, 17.9 mL, 0.19 mol) was then added in one portion. The reaction mixture was stirred for 7 hours at the same temperature. Solid salts were filtered and washed thoroughly with dichloromethane. To remove the catalyst the filtrate was passed through a pad of silica (10 mm $\times$ 50 mm) eluting with dichloromethane. The solvent was evaporated, and the resulting yellowish residue distilled in vacuo affording (*R*)-2-chiorobenzaidehyde cyanohydrin acetate. Yield 8.87 g (88.6%); b.p. 127-130°C (0.2 mm); ee (R), 88.3%; $[\alpha]^{25}_{D}$ (c=1, in $CHCl_3$); $n^{25}_{D}$ 1.51189; $^1H$ NMR (200 MHz, $CDCl_3$, 25°C): $\delta$ = 2.15 (s, 3H; $CH_3$); 6.66 (s, 1 H; CH); 7.32-7.70 (m, 4H; ArH). Elemental analysis (%) calculated for $C_{10}H_8ClNO_2$: C 57.30, H 3.85, Cl 16.91, N 6.68; found C 56.93, H 3.83, Cl 17.03, N 6.69.

The procedure was repeated using different aldehydes and catalysts. Details of the aldehydes and catalysts employed, and the results obtained, are given in Table 2 below. Chemical yields were measured by NMR, unless specified

otherwise.

Table 2. Enantioselective synthesis of *O*-acetyl cyanohydrins, according to Scheme 2 promoted by **1 a** or 1 **b**,(a)

| Aldehyde | Catalyst | ChemicalYield,% | Enantiomeric Excess,ee %[c] Configuration |
|---|---|---|---|
| PhCHO | **1a[1b]** | 93 [92[d]] | 90(*S*) [89(*R*)] |
| *p*-$MeOC_6H_4CHO$ | **1b** | 74 | 93*(R)* |
| *m*-$MeOC_6H_4CHO$ | **1b** | [99] | 93*(R)* |
| *m*-$PhOC_6H_4CHO$ | **1a[1b]** | 99 [99] | 90*(S)* [89*(R)*] |
| *p*-$FC_6H_4CHO$ | **1a[1b]** | 98 [99] | 92*(S)* [93*(R)*] |
| *o*-$FC_6H_4CHO$ | **1a[1b]** | 87[86] | 85*(S)* [82*(R)*] |
| *m*-$FC_6H_4CHO$ | **1b** | 99 | 89*(R)* |
| *o*-$ClC_6H_4CHO$ | **1a[1b]** | 87 [89[d]] | 86*(S)* [88*(R)*] |
| $PhCH_2CH_2CHO$ | **1a[1b]** | 80 [79[d]] | 84*(S)* [82*(R)*] |
| $(CH_3)_2CHCHO$ | **1a[1b]** | 64 [62[d]] | 69*(S)* [72*(R)*] |
| $(CH_3)_3CCHO$ | **1a[1b]** | 40 [40[d]] | 62*(S)* [60*(R)*] |

[c] Determined by chiral GLC. [d] Yield of isolated product.

## Claims

1. A process for cyanating an aldehyde which comprises reacting the aldehyde with:

   i) a cyanide source which does not comprise a Si-CN or a C-(C=O)-CN moiety; and
   ii) a substrate having the general formula Q-Y where Q represents an organic acid radical selected from the group comprising: R-(C=O)-, R-(C=S)-, RO-(C=O)-, RN-(C=O)-, RO-(C=S)-, RN-(C=S)-, RS-(C=O)-, RS-(C=S)-, R-(P=O)(OR)-, $R-SO_2$- and R-SO-, wherein R represents a substituted or unsubstituted hydrocarbyl group; and Y represents a non-halogen leaving group; in the presence of a catalyst comprising a chiral complex of titanium or vanadium.

2. A. process according to claim 1, in which the cyanide source is an alkali metal cyanide.

3. A process according to claim 2, in which the cyanide source is potassium cyanide.

4. A process according to claims 1, 2 or 3 in which Q-Y is a carboxylic anhydride or carbonic acid anhydride.

5. A process according to any one of claims 1 to 4 wherein the catalyst is a transition metal complex comprises a chiral ligand wherein the chiral ligand is a binol, taddol, sulfoximine, salicylimine, tartrate or Salen ligand.

6. A process according to any of claims 1 to 5, wherein the catalyst is of the general formula

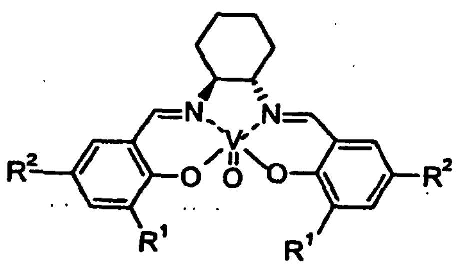

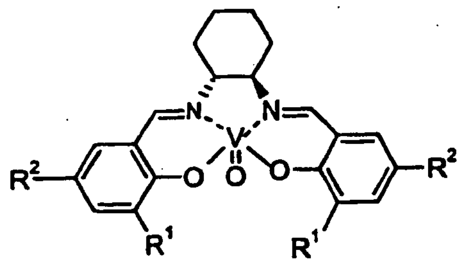

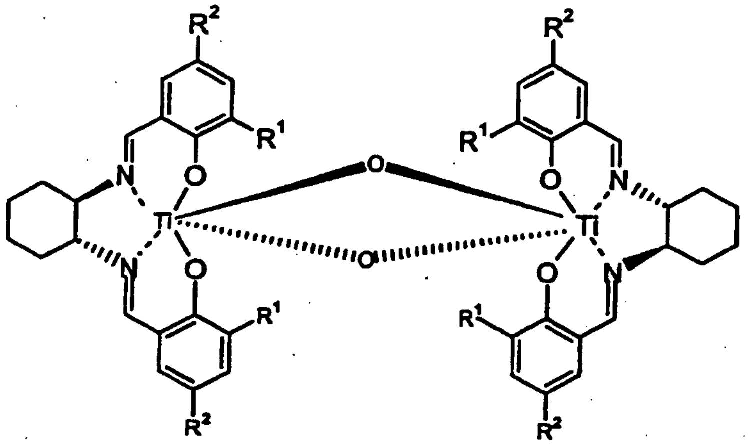

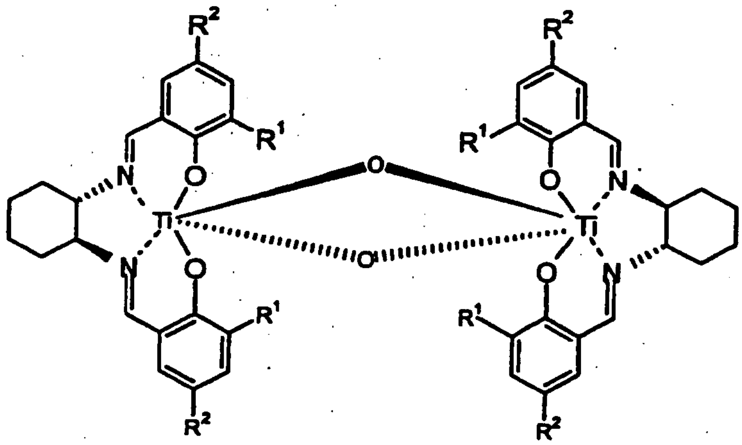

wherein each $R^1$ and $R^2$ are independently H, alkyl, aryl, aralkyl, alkoxy, aryloxy, halogen, nitro, halo-oalkyl, amino or amido.

**7.** A process according to any one of claims 1 to 6 wherein the process is carried out in the presence of an additive having a pKa of greater than 10.

**8.** A process according to claim 7, wherein the additive is selected from pyridine, 2,6-lutidine, imidazole, t-butanol; and water.

**9.** A process according to any one of claims 1 to 8 wherein the process is carried out in a polar, aprotic solvent.

**10.** A process according to any of claims 1 to 9 wherein Q-Y is added to a mixture of catalyst, cyanide source, aldehyde, polar aprotic solvent and additive having a pKa of greater than 10.

**11.** A process according to claim 1 for the cyanation of an aldehyde group which comprises reacting the aldehyde with:

i) an alkali metal cyanide; and
ii) a carboxylic anhydride;

in the presence of a catalyst.

**12.** A process according to claim 1 for the preparation of an O-acyl cyanohydrin which comprises reacting an aldehyde with potassium cyanide and a carboxylic anhydride in the presence of a catalyst.

**13.** A process according to claim 11 or 12, wherein the chiral complex of titanium or vanadium has the formula:

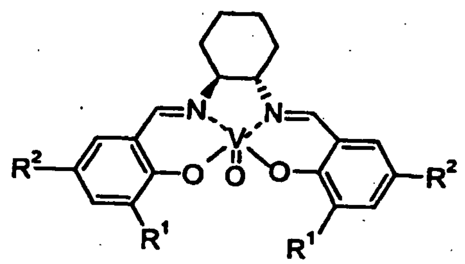

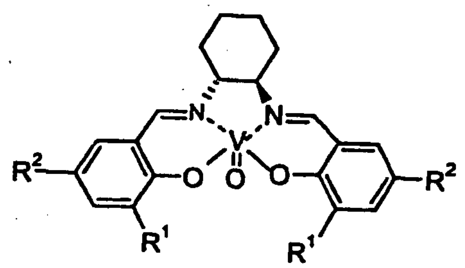

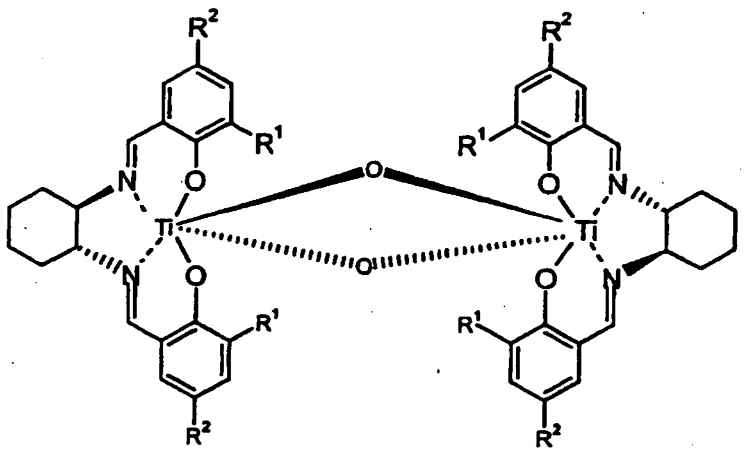

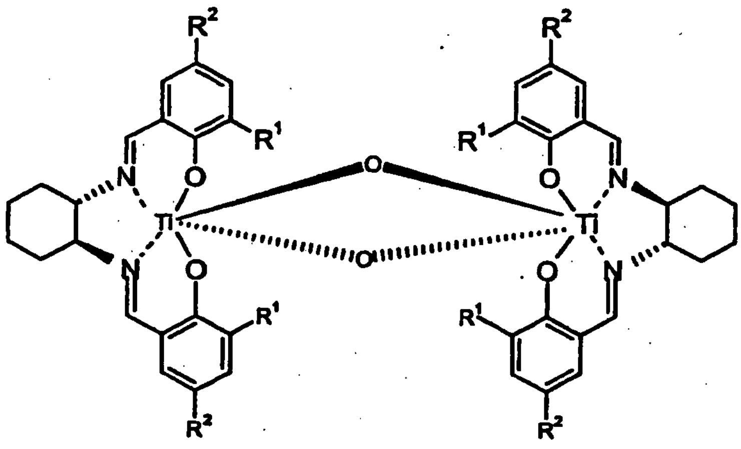

wherein each $R^1$ and $R^2$ are independently H, alkyl, aryl, aralkyl, alkoxy, aryloxy, halogen, nitro, halo-oalkyl, amino or amido.

**Patentansprüche**

1. Verfahren zur Cyanierung eines Aldehyds, umfassend die Umsetzung des Aldehyds mit:

   i) einer Cyanidquelle, welche keinen Si-CN oder C-(C=O)-CN-Rest enthält; und
   i) einem Substrat mit der allgemeinen Formel Q-Y, wobei Q einen organischen Säurerest darstellt, ausgewählt aus der Gruppe umfassend: R-(C=O)-, R-(C=S)-, RO-(C=O)-, RN-(C=O), RO-(C=S)-, RN-(C=S)-, RS-(C=O)-, RS- (C=S) -, R-(P=O)(OR)-, R-$SO_2$- und R-SO-, worin R eine substituierte oder unsubstituierte Hydrocarbylgruppe darstellt; and Y eine Nichthalogenabgangsgruppe darstellt; in Gegenwart eines Katalysators, der einen chiralen Komplex mit Titan oder Vanadium umfasst.

2. Verfahren gemäß Anspruch 1, in welchem die Cyanidquelle ein Alkalimetallcyanid ist.

3. Verfahren gemäß Anspruch 2, in welchem die Cyanidquelle Kaliumcyanid ist.

4. Verfahren gemäß den Ansprüchen 1, 2 oder 3, in welchem Q-Y ein Carbonsäureanhydrid oder ein Kohlensäureanhydrid ist.

5. Verfahren gemäß irgendeinem der Ansprüche 1 bis 4, wobei es sich bei dem Katalysator um einen Übergangsmetallkomplex handelt der einen chiralen Liganden enthält, wobei der chirale Ligand ein Binol-, Taddol-, Sulfoximin-, Salicylimin-, Tartrat- oder Salenligand ist.

6. Verfahren gemäß irgendeinem der Ansprüche 1 bis 5, wobei der Katalysator die allgemeine Formel

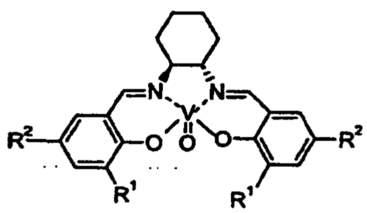

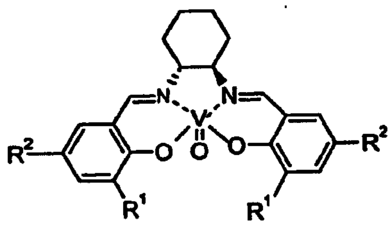

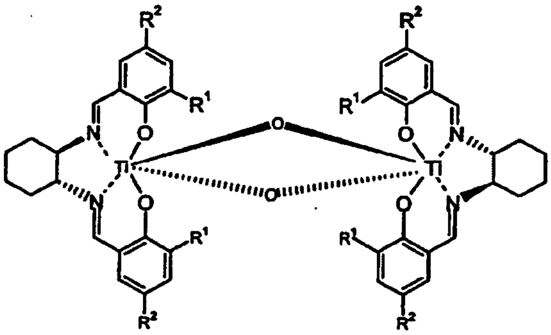

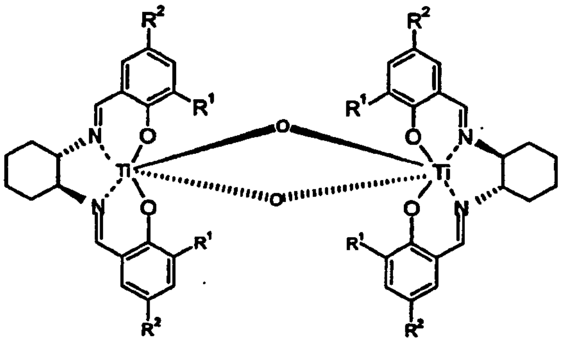

besitzt, worin $R^1$ and $R^2$ unabhängig voneinander H, Alkyl, Aryl, Aralkyl, Alkoxy, Aryloxy, Halogen, Nitro, Halo-O-Alkyl, Amino oder Amido sind.

**7.** Verfahren gemäß irgendeinem der Ansprüche 1 bis 6, worin das Verfahren in Gegenwart eines Zusatzstoffes mit einem pKa-Wert größer als 10 durchgeführt wird.

**8.** Verfahren gemäß Anspruch 7, worin der Zusatzstoff ausgewählt ist aus Pyridin, 2,6-Lutidin, Imidazol, t-Butanol; und Wasser.

**9.** Verfahren gemäß irgendeinem der Ansprüche 1 bis 8, worin das Verfahren in einem polaren, aprotischen Lösemittel durchgeführt wird.

**10.** Verfahren gemäß irgendeinem der Ansprüche 1 bis 9, worin Q-Y zu einer Mischung aus Katalysator, Cyanidquelle, Aldehyd, polarem aprotischen Lösemittel und Zusatzstoff mit einem pKa-Wert größer als 10 hingefügt wird.

**11.** Verfahren gemäß Anspruch 1 zur Cyanierung einer Aldehydgruppe, umfassend die Umsetzung des Aldehyds mit:

i) einem Alkalimetallcyanid; und
ii) einem Carbonsäureanhydrid;

in Gegenwart eines Katalysators.

**12.** Verfahren gemäß Anspruch 1 zur Herstellung eines O-Acylcyanhydrins, welches die Umsetzung eines Aldehyds mit Kaliumcyanid und einem Carbonsäureanhydrid in Gegenwart eines Katalysators umfasst.

**13.** Verfahren gemäß Anspruch 11 oder 12, wobei der chirale Komplex mit Titan oder Vanadium die allgemeine Formel:

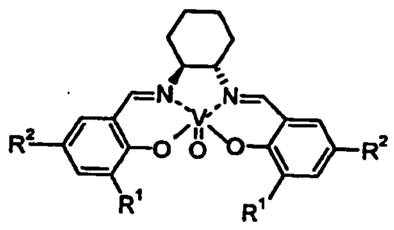

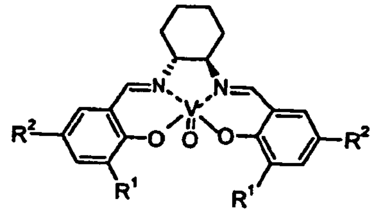

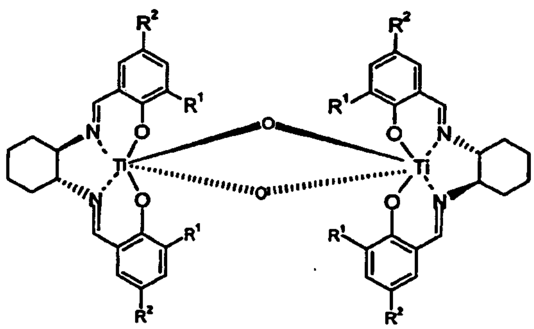

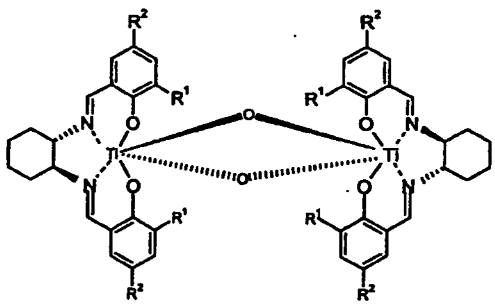

besitzt, worin $R^1$ and $R^2$ unabhängig voneinander H, Alkyl, Aryl, Aralkyl, Alkoxy, Aryloxy, Halogen, Nitro, Halo-O-Alkyl, Amino oder Amido sind.

## Revendications

1. Procédé de transformation d'un aldéhyde en cyanate qui comprend la réaction de l'aldéhyde avec :

   i) une source de cyanure qui ne comprend pas un Si-CN ou une moitié C-(C=O)-CN ; et
   ii) un substrat présentant la formule générale Q-Y dans laquelle Q représente un radical d'acide organique choisi parmi : R-(C=O)-, R-(C=S)-, RO-(C=O)- RN-(C=O)-, -RO-(C=S)-, RN-(C=S)-, RS-(C=O)-, RS-(C=S)-, R-(P=O)(OR)-, R-$SO_2$- et R-SO-, où R représente un groupe hydrocarbyle substitué ou non substitué ; et Y représente un groupe qui s'éloigne non-halogène ; en présence d'un catalyseur comprenant un complexe chiral de titane ou de vanadium.

2. Procédé selon la revendication 1, dans lequel la source de cyanure est un cyanure de métal alcalin.

3. Procédé selon la revendication 2, dans lequel la source de cyanure est le cyanure de potassium.

4. Procédé selon les revendications 1, 2 ou 3, dans lequel Q-Y est un anhydride carboxylique ou un anhydride d'acide carbonique.

5. Procédé selon l'une quelconque des revendications 1 à 4, dans lequel le catalyseur est un complexe de métal de transition qui comprend un ligand chiral dans lequel le ligand chiral est un binol, un taddol, une sulfoximine, une salicylimine, un tartrate ou un ligand Salen.

6. Procédé selon l'une quelconque des revendications 1 à 5, dans lequel le catalyseur est de la formule générale

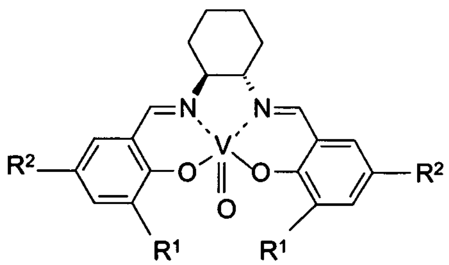

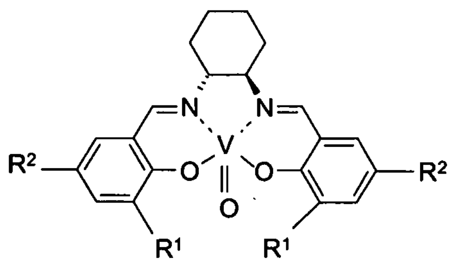

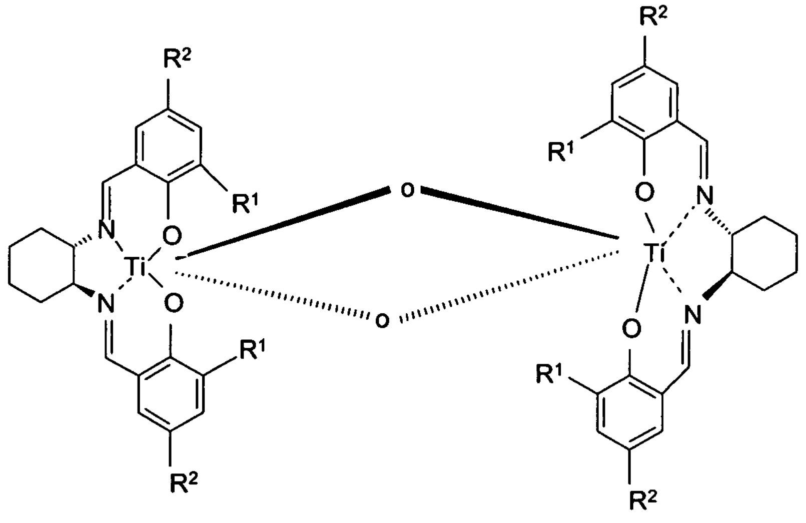

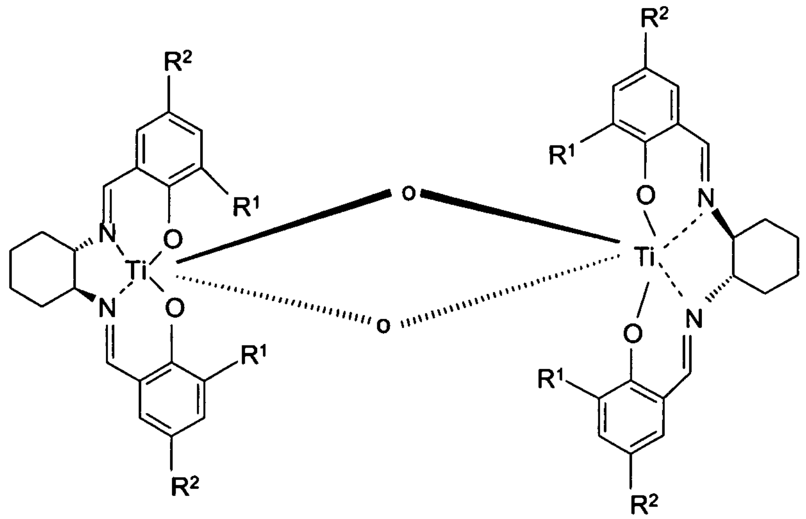

dans lesquelles chaque $R^1$ et $R^2$ est indépendamment H, un groupe alkyle, aryle, aralkyle, alcoxy, aryloxy, un atome d'halogène, un groupe nitro, halo-o-alkyle, amino ou amido.

**7.** Procédé selon l'une quelconque des revendications 1 à 6, dans lequel le procédé est réalisé en présence d'un additif présentant un pKa supérieur à 10.

**8.** Procédé selon la revendication 7, dans lequel l'additif est choisi parmi la pyridine, la 2,6-lutidine, l'imidazole, le t-butanol et l'eau.

**9.** Procédé selon l'une quelconque des revendications 1 à 8, dans lequel le procédé est réalisé dans un solvant polaire aprotique.

**10.** Procédé selon l'une quelconque des revendications 1 à 9, dans lequel Q-Y est ajouté à un mélange de catalyseur, de source de cyanure, d'aldéhyde, de solvant aprotique polaire et d'additif présentant un pKa supérieur à 10.

**11.** Procédé selon la revendication 1 pour la transformation d'un groupe aldéhyde en cyanate qui comprend la réaction de l'aldéhyde avec :

i) un cyanure de métal alcalin ; et

ii) un anhydride carboxylique ;

en présence d'un catalyseur.

**12.** Procédé selon la revendication 1 pour la préparation d'une O-acylcyanohydrine qui comprend la réaction d'un aldéhyde avec du cyanure de potassium et un anhydride carboxylique en présence d'un catalyseur.

**13.** Procédé selon la revendication 11 ou 12, dans lequel le complexe chiral du titane ou du vanadium présente la formule :

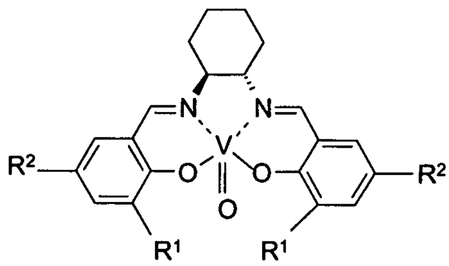

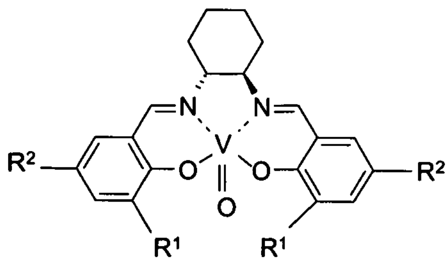

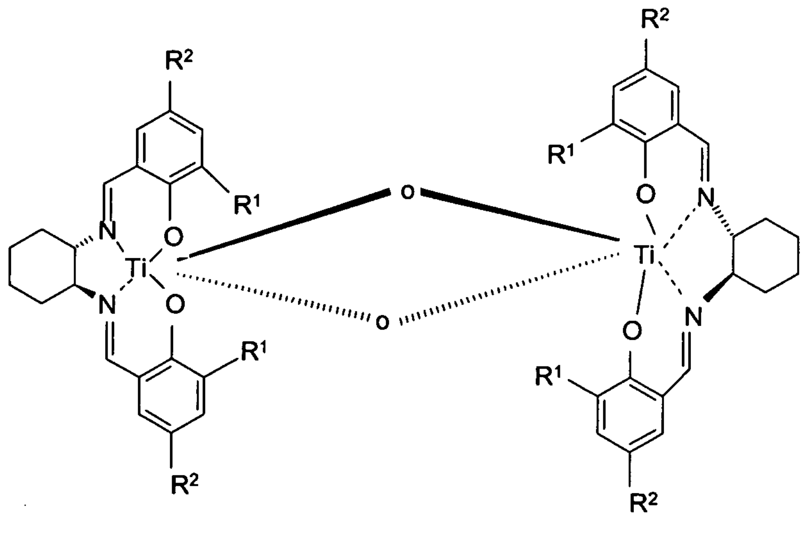

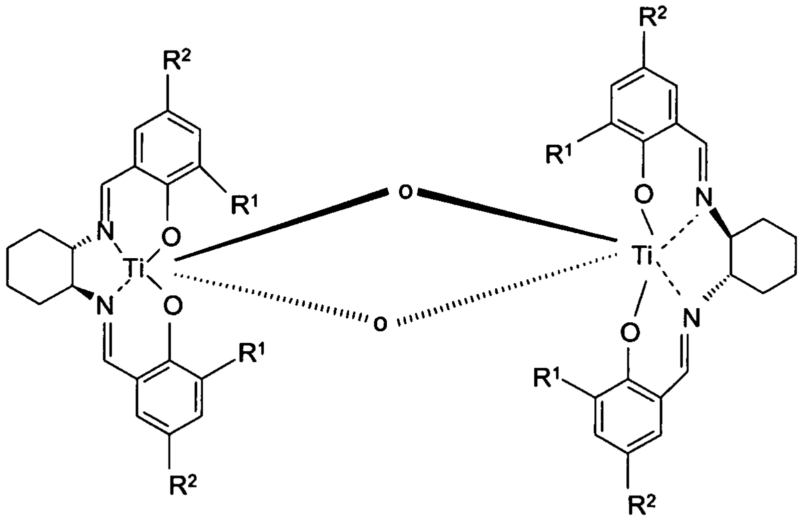

dans lesquelles chaque $R^1$ et $R^2$ est indépendamment H, un groupe alkyle, aryle, aralkyle, alcoxy, aryloxy, un atome d'halogène, un groupe nitro, halo-o-alkyle, amino ou amido.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7070040 A
- JP 6271522 A


### Non-patent literature cited in the description


- **M.NORTH.** *Synlett,* 1993, 807-20
- **F.EFFENBERGER.** *Angew. Chem. Int. Ed, Engl.,* 1994, vol. 33, 1555
- **M.NORTH ; KATRITZKY, A.R. ; METH-COHN, O. ; REES, C.W. ; PATTENDEN. G.** Comprehensive Organic Functional Group Transformations. Pergamon Press, 1995, vol. 3
- **Y BELOKON et al.** *Tetrahedron Asymmetry,* 1996, vol. 7, 851-5
- **Y.BELOKON et al.** *J.Chem. Soc., Perkin Trans.,* 1997, vol. 1, 1293-5
- **Y.N.BELOKON et al.** Izvestiya Akademii Nauk. *Seriya Khimicheskaya,* 1997, 2040
- *Russian Chem. Bull.,* 1997, vol. 46, 1936-8
- **V.I.TARAROV et al.** *Chem. Commun.,* 1998, 387-8
- **Y.N.BELOKON et al.** *J. Am. Chem. Soc.,* 1999, vol. 121, 3958-73
- **V.I.TARAROV et al.** *Russ. Chem. Bull.,* 1999, vol. 48, 1128-30
- **Y. N.BELOKON et al.** *Tetrahedron Lett.,* 1999, vol. 40, 8147-50
- **Y.N.BELOKON et al.** *Eur. J. Org. Chem.,* 2000, 2655-61
- **Y.N.BETOKON ; M.NORTH ; T.PARSONS.** *Org. Lett.,* 2000, vol. 2, 1617-9
- *J. Am. Chem. Soc.,* 1999, vol. 121, 3968-73